# EUROPÄISCHE PATENTSCHRIFT

(11) **EP 1 149 173 B1**
(45) Veröffentlichungstag und Bekanntmachung des Hinweises auf die Patenterteilung: **22.08.2012**
(21) Anmeldenummer: 00912349.8
(22) Anmeldetag: 28.01.2000
(51) Int. Cl.: C12Q 1/56

(54) **VERFAHREN ZUR BESTIMMUNG DER KONZENTRATION VON THROMBININHIBITOREN**
METHOD FOR DETERMINING THE CONCENTRATION OF THROMBIN INHIBITORS
PROCEDE PERMETTANT DE DETERMINER LA CONCENTRATION D'INHIBITEURS DE THROMBINE

(30) Priorität: 04.02.1999 DE 19904674
(43) Veröffentlichungstag der Anmeldung: 31.10.2001
(73) Patentinhaber: JenAffin GmbH, 07745 Jena (DE)
(72) Erfinder: NOWAK, Götz, D-07747 Jena (DE); BUCHA, Elke, D-99094 Erfurt (DE)
(74) Vertreter: Jungblut, Bernhard Jakob
(86) Internationale Anmeldenummer: PCT/DE2000/000330
(87) Internationale Veröffentlichungsnummer: WO 2000/046602

(56) Entgegenhaltungen:
- EP-A- 0 570 355
- US-A- 5 529 905
- US-A- 5 547 850
- HAN JIN-HUA ET AL: "Inhibition of meizothrombin and meizothrombin(desF1) by heparin cofactor II." JOURNAL OF BIOLOGICAL CHEMISTRY, Bd. 272, Nr. 45, 7. November 1997 (1997-11-07), Seiten 28660-28665, XP002141790 ISSN: 0021-9258
- DOYLE M F ET AL: "MULTIPLE ACTIVE FORMS OF THROMBIN IV. RELATIVE ACTIVITIES OF MEIZOTHROMBINS" JOURNAL OF BIOLOGICAL CHEMISTRY, Bd. 265, Nr. 18, 1990, Seiten 10693-10701, XP002141791 ISSN: 0021-9258

## Beschreibung

Die Erfindung betrifft ein Verfahren zur Bestimmung der Konzentration von Thrombininhibitoren, wobei der Körperflüssigkeit eines Lebewesens eine Prothrombin in Meizothrombin bzw. Meizothrombin-des Fragment 1 (folgend MTdesfg1)spaltende Substanz zugegeben wird. - Als Thrombininhibitoren werden alle natürlichen oder synthetische Stoffe verstanden, die Thrombin oder Thrombinvorläufer direkt inhibieren. Als Beispiel für einen natürlichen Thrombininhibitor ist Hirudin zu nennen, welches aus dem Speichel von Hirudo medicinalis gewonnen werden kann. Hirudin ist ein sehr kleines Protein bestehend aus 65 Aminosäuren und mit einem Molekulargewicht von 7 kD. Beispiele für synthetische Thrombininhibitoren sind die sogenannten Hirologe, welche dem Hirudin analoge bzw. homologe Teilsequenzen aufweisen, sowie Polypeptide bestehend aus oder mit einem Tripeptid Phe-Pro-Arg oder Derivaten eines solchen Tripeptids, wie beispielsweise Borsäurederivate, Chloromethylketonderivate, Benzamidinderivate, Argininale, aminosäuremodifizierte Derivate und dergleichen. Den vorstehenden Substanzen ist höchstwahrscheinlich der im wesentlichen gleiche Wirkmechanismus wie bei Hirudin gemeinsam. Als Spenderlebewesen für die Körperflüssigkeit kommen Menschen und Säugetiere, wie beispielsweise Rodenten, in Frage. Beispiele für Körperflüssigkeiten sind insbesondere Blut bzw. aus Blut hergestelltes Blutplasma. Aber auch Körperflüssigkeiten, welche kein Prothrombin enthalten, kommen in Frage, wie z.B. Urin, Liquor, Speichel, Peritonaealflüssigkeit u.a. Dann wird im Rahmen der Erfindung Prothrombin zugesetzt. Nicht-trüb meint, daß keine beachtlichen Mengen an Schwebstoffen in der zu untersuchenden Körperflüssigkeit vorliegen sollen. Dies kann erforderlichenfalls beispielsweise durch Zentrifugation der Körperflüssigkeit und Abzug des überstandes erreicht werden.

Der der Erfindung zugrundeliegende theoretische Hintergrund ist der folgende. Die Umwandlung von Prothrombin in Thrombin ist ein wesentlicher Faktor in der Blutgerinnung. Thrombin wirkt auf die Bildung von Fibrinmonomeren aus Fibrinogen sowie auf die Polymerisation der Fibrinmonomere. Prothrombin wird in Thrombin umgewandelt unter Mitwirkung von aktiviertem Faktor x, aktiviertem Faktor V Ca⁺⁺-Ionen und Phospholipiden, wie z.B. Plättchenfaktor 3. Hierbei findet eine mehrstufige Reaktion statt, wobei Intermediate in vergleichbar geringer Menge gebildet werden. Wenn jedoch die Koagulation mittels beispielsweise Ecarin oder einem anderen Schlangengift bzw. Schlangengiftfraktion eingeleitet wird, so entsteht demgegenüber ein "typisches" Intermediat, beispielsweise Meizothrombin, PIVKA Meizothrombin oder Meizothrombin-des Fragment-1 (PIVKA ist die Abkürzung für ein Protein, welches durch einen Vitamin K Antagonisten induziert wird). Diese atypischen Intermediate werden interessanterweise durch beispielsweise Hirudin inaktiviert, nicht jedoch durch Heparin (Inhibitor der Faktoren IIa, IXa, XIa, XIIa und/oder Antithrombin). Sie führen im übrigen ebenfalls zur Thrombinbildung und subsequent zur Gerinnung. Diese Eigenschaft der "atypischen" Intermediate Faktoren der Blutgerinnungskaskade aktivieren zu können, ist in Doyle et al, The Journal of Biological Chemistry, vol. 265, No. 18 pp 10693 - 10701, Jun. 1990, EP 0 570 355 A1 und US 5,529,905 beschrieben. Die Affinität von Hirudin und anderen synthetischen Thrombininhibitoren zu den atypischen Intermediaten ist sehr hoch (k₁ > 10⁻¹⁰ mol/l für Meizothrombin), so daß freies atypisches Intermediat von dem Thrombininhibitor kurzfristig gebunden wird. Die Hemmung von Prothrombinfragmenten durch Thrombininhibitoren ist in Jin-Hua, Han et al., The Journal of Biological Chemistry, vol. 272, No. 45 pp 28660 - 28665, Nov. 1997 dargelegt.

Die vorstehenden Zusammenhänge werden in einem Verfahren der eingangs genannten Art, welches beschrieben ist in der Literaturstelle US-A-5,547,850, genutzt, wobei gleichsam der Verbrauch des Thrombininhibitors durch Messung der Verzögerung der Gerinnung erfaßt wird. Eine große Menge an Thrombininhibitor führt zu einer langen Zeit bis zum Gerinnungseintritt und umgekehrt. Dieses verfahren hat sich in der Praxis grundsätzlich ausgezeichnet bewährt. Als nachteilig hat sich jedoch erwiesen, daβ in Fallen verminderten Fibrinogenspiegels Verfälschungen auftreten können, da ein (zu) geringer Fibrinogenspiegel ebenso wie ein hoher Thrombininhibitorspiegel zu langen Gerinnungszeiten führen kann.

Der Erfindung liegt das technische Problem zugrunde, ein Verfahren zur Bestimmung der Konzentration von Thrombininhibitoren anzugeben, welches unabhängig vom Fibrinogenspiegel genaue Werte liefert.

Zur Lösung dieses Problems lehrt die Erfindung ein Verfahren zur Bestimmung der Konzentration von Thrombininhibitoren in einer nicht-trüben Körperflüssigkeit oder einem nicht-trüben Extrakt aus einer Körperflüssigkeit mit den folgenden Verfahrensschritten: a) die Körperflüssigkeit eines Lebewesens wird erforderlichenfalls einer Abtrennung von Trübstoffen unterworfen, b) der in Stufe a) erhaltenen nicht-trüben Körperflüssigkeit werden ein nicht in die Umwandlung prothrombin/aktives Meizothrombin bzw. Mtdesfgl eingreifendes gerinnungshemmendes Mittel, ein durch aktives Meizothrombin bzw. Mtdesfgl spaltbares chromogenes oder fluorogenes Substrat und eine Prothrombin in Meizothrombin bzw. Mtdesfgl spaltende Substanz zugegeben, sowie, optional, Prothrombin, c) die in Stufe b) erhaltene Lösung bzw. Mischung wird einer wellenlängenselektiven Lichtabsorptions- oder Lichtemissionsmessung in Abhängigkeit von der Zeit unterworfen, d) aus der Verminderung der Lichtabsorption oder Lichtemission in Stufe c) je Zeiteinheit wird die in der Körperflüssigkeit enthaltene Menge des Thrombininhibitors durch Vergleich mit ermittelten Standardkurven bestimmt. Alternativ zur Prothrombin in Meizothrombin bzw. Mtdesfgl spaltenden Substanz oder ergänzend kann Meizothrombin bzw. Mtdesfg1 zugegeben sein. . -Als chromogenes Substrat werden Substanzen bezeichnet, welche chromophore Gruppen enthalten und spezifisch von Thrombin farbgebend gespalten werden. Fluorogene Substrate sind Substanzen, welche spezifisch von Thrombin unter Bildung von fluoreszierenden Substanzen spaltbar sind. Prothrombin kann zugesetzt werden, wenn die Körperflüssigkeit nicht natürlicherweise ausreichend Prothrombin enthält, wie beispielsweise im Falle von Vitamin K Mangel, oder wenn die zu erwartende Menge an Thrombininhibitor oder Aktivität des Thrombininhibitors dies empfiehlt, oder wenn während einer Krankheit ein Prothrombin-Mangel aufgetreten ist.

Die Erfindung beruht auf der überraschenden Erkenntnis, daß chromogene bzw. fluorogene Substanzen, welche spezifisch von Thrombin gespalten werden, ebenso spezifisch von Meizothrombin bzw. Mtdesfg1 spaltbar sind. Dies ist nicht zu erwarten, da Intermediate zwar notwendige Vorstufen darstellen, jedoch natürlicherweise nicht dieselbe Wirkungen bzw. Reaktivitäten wie das Thrombin entfalten. Dadurch, daß die erfindungsgemäße Nachweisreaktion allein durch die Überwachung der Meizothrombin bzw. Mtdesfg1-Inhibierung mittels einer Farbreaktion erfolgt, ist der Nachweis völlig unabhängig von dem Fibrinogenspiegel. Vielmehr muß beim Einsatz von Körperflüssigkeiten, insbesondere Blut bzw. Blutplasma, die Gerinnung sogar unterbunden werden, um die Farbreaktionsauswertung nicht zu stören. Zudem ist die erfindungsgemäße Thrombininhibitorbestimmung in allen Bereichen mindestens ebenso genau, wie die Bestimmung mittels der vorbekannten Methode bei hohem Fibrinogenspiegel. Auch besteht Unabhängigkeit von eventuell in der Körperflüssigkeit enthaltenen, oral verabreichten Antikoagulantien. Weitere Vorteile sind: schnelle Messung innerhalb von Minuten in chromogenen Kanälen üblicher Gerinnungsautomaten (diese messen eine Trübung oft bei mehreren Wellenlänge zwecks Korrektur und weisen daher in der Regel die Möglichkeit zur wellenlängenselektiven und wellenlängenvariablen Lichtabsorptionsmessung auf); hohe Reproduzierbarkeit der gefundenen Werte aufgrund einer sehr niedrigen Streuung der Einzelwerte (das Konfidenzintervall liegt gemäß einer Vielzahl von Versuchsserien unter 5%, in der Regel bei 2,2 - 3,5%); die hohe Genauigkeit bzw. Reproduzierbarkeit wird zudem auch bei sehr hohen Thrombininhibitor- bzw. Hirudinspiegeln erreicht; aufgrund der vorstehenden Eigenschaften eignet sich das erfindungsgemäß Verfahren zur nationalen und internationalen Standardisierung.

Das erfindungsgemäße Verfahren findet Einsatz einerseits in der Wissenschaft, nämlich in allen Bereichen von Untersuchungen, in denen Thrombininhibitorkonzentrationen bestimmt werden müssen, sowie dem (ggf. high capacity) Screenen von prospektiven Thrombininhibitoren. In letzterem Fall kann mit hohem Durchsatz eine Vielzahl von synthetischen prospektiven Inhibitoren auf ihre tatsächliche Wirkung untersucht werden. Aktivität meint hierbei die Feststellung, ob überhaupt eine Inhibierung stattfindet und bejahendenfalls, wie die Kinetik bzw. spezifische Aktivität ist. Andererseits bietet sich auch der klinische Einsatz an, beispielsweise bei der Überwachung von Thrombininhibitorspiegeln bei Patienten, welchen der Inhibitor aus therapeutischen Gründen verabreicht wird. Auf einfache und kostengünstige Weise kann so vermieden werden, daß eine Unter- oder Überdosierung des Thrombininhibitors stattfindet, und zwar sowohl in quasi-kontinuierlicher oder diskontinuierlicher Überwachung.

Im einzelnen kann das nicht in die Umwandlung prothrombin/aktives Meizothrombin bzw. Mtdesfgl eingreifende gerinnungshemmende Mittel ausgewählt sein aus der Gruppe "Calcium-Komplexbildner, Heparin, Heparinoide, Antithrombin III, Protein C, Fibrinpolymerisationshemmstoffe und Mischungen aus diesen Stoffen". Ein konkretes Beispiel hierfür ist Pefabloc FG der Firma Pentapharm AG, Basel, Schweiz, welches ein Tetrapeptid (Gly-Pro-Arg-Pro) ist und mit hoher Affinität die Fibrinogen-Polymerisation verhindert. Die Prothrombin in Meizothrombin bzw. Mtdesfg1 spaltende Substanz kann ausgewählt sein aus der Gruppe der Schlangengifte und Schlangengiftfraktionen, beispielsweise Gifte von Dispholidus, Rhabdophis, Bothrops, Notechis, Oxyuranus und Russel Vipern. Zweckmäßigerweise werden gereinigte Fraktionen daraus verwendet. Vorzugsweise wird Ecarin, eine hochgradig gereinigte Fraktion des Echis-carinatus Toxins oder Multisquamase, das Prothrombin-spaltende Enzym aus Echis multisquamatous, verwendet. Solche Substanzen, wie beispielsweise Ecarin sind käuflich erwerbar u.a. von der Firma Pentapharm AG, Schweiz.

Das durch aktives Meizothrombin bzw. Mtdesfg1 Spaltbare chromogene Substrat kann unter Spaltung p-Nitroanilin freisetzen und die Lichtabsorptionsmessung kann dann bei 405 nm durchgeführt werden. Beispiele für solche oder auch andere Substrate sind Tripeptide, welche unter den Namen Chromozym TH oder Pefachrom TH von den Firmen Chromogenix, Boehringer, Pentapharm erhältlich sind (Pefachrome TH ist H-D-ChG-Ala-Arg-pN.2ACOH). Ein Beispiel für fluorochrome Substrate ist Pefachrom TH fluorogen, welches unter den Namen Pefa 15865 von der Firma Pentapharm erhältlich sind.

Im einzelnen empfiehlt es sich bei den in Frage kommenden Aktivitäten, in Stufe c) eine erste Absorptions- oder Emissionsmessung nach 0 - 100 s, vorzugsweise 0 - 50, höchsvorzugsweise 5 - 15 s, und eine zweite nach anschließenden 10 - 1000 s, vorzugsweise 50 - 500 s, höchstvorzugsweise 150 - 300 s, gezählt ab Zugabe der Prothrombin in Meizothrombin bzw. Mtdesfgl spaltenden Substanz oder des Meizothrombins bzw. MTdesfg1, durchzuführen. Das erfindungsgemäße Verfahren ist insbesondere zur Bestimmung vom Hirudin oder der Bestimmung der Konzentration und/oder der Aktivität von synthetischen Thrombininhibitoren oder Hirulogen.

Die Erfindung betrifft weiterhin ein Test Kit zur Bestimmung der Konzentration von Thrombininhibitoren in einer nicht-trüben Körperflüssigkeit oder einem nicht-trüben Extrakt aus einer Körperflüssigkeit mit folgenden Kitkomponenten: K1) einer Lösung eines nicht in die Umwandlung Prothrombin/aktives Meizothrombin bzw. Mtdesfg1 eingreifenden gerinnungshemmenden Mittel, K2) einem durch aktives Meizothrombin bzw. Mtdesfg1 spaltbaren chromogenen oder fluorogenen Substrat und K3) einer Lösung einer Prothrombin in Meizothrombin bzw. Mtdesfg1spaltenden Substanz, wobei die Komponente K3) ersetzt oder ergänzt sein kann durch eine Komponente K3a) einer Lösung mit Meizothrombin bzw. Mtdesfg1. Die Kitkomponenten können voneinander getrennt aber in einer einzigen Textkitpackung vorgesehen sein. Es kann als optional einsetzbare zusätzliche Kitkomponente eine Lösung mit Prothrombin vorgesehen ist.

In jedem Fall versteht sich, daß bei Zugabe von Thrombin, Meizothrombin bzw. Mtdesfgl und/oder Meizothrombin bzw. Mtdesfg1 spaltenden Substanzen diese in definierten, vorgegebenen Mengen eingesetzt werden. Entsprechendes gilt für Substrat.

Für das erfindungsgemäße Test Kit gelten die zum erfindungsgemäßen Verfahren getroffenen Detailerläuterungen entsprechend.

Sofern Meizothrombin bzw. Mtdesfg1 eingesetzt wird, so kann dies käuflich erworben werden, beispielsweise von der Fa. Pentapharm AG, Schweiz, aber auch beispielsweise gemäß der Vorschrift in der Literaturstelle US-A-5,547,850 hergestellt werden an immobilisiertem Ecarin.

Bei den im Rahmen der Erfindung einsetzbaren Geräten handelt es beispielsweise um meist ohnehin vorhandene halb- oder vollautomatische Gerinnungsgeräte. In Frage kommen dabei z.B. Gerinnungsautomaten des Typs Sysmex CA-500 oder S2000 der Firma Dade-Behring oder des Typs Electra 2000. Beim CA-500 wird das von einer LED emittierte Licht durch einen Filter (405nm) gesandt und anschließend durch die Probe. Das CA-500 bestimmt im chromogenen Kanal die Änderung bzw. Verminderung der Lichtabsorption von Farbstoffen, wie z.B. pNA (p-Nitroanilin). Ist in einer Probe beispielsweise Hirudin, so wird das generierte oder zugegebene Meizothrombin bzw. Mtdesfg1 inaktiviert mit der Folge einer dadurch behinderten pNA-Freisetzung. Die insofern sich anders verhaltende (ändernde) optische Dichte der Probe wird mittels einer Photodiode aufgenommen und ausgewertet. Die zu beobachtende Lichtabsorptionsänderung ist umgekehrt proportional der Hirudinaktivität.

Im folgenden wird die Erfindung anhand von lediglich Ausführungsbeispiele darstellenden Experimenten näher erläutert.

Zur Bestimmung einer Standardkurve wurde gepooltes Humancitratplasma mit vorgegebenen Mengen Hirudinlösung versetzt. Die so erhaltenen Standardlösungen wurden in einem Ca-500 gemessen.

Als Reagenzien wurden eingefüllt,
Reagent 1 [Inhib] (Raumtemperatur): 400ul Pefabloc FG (20 mM; gelöst in 0,9% NaCl) + 2100 µl Tris-Puffer,
Reagent 2 [Chromo] (Raumtemperatur): Pefachrome TH (10µmol/vial), verdünnt auf 3µmol/ml Aq. dest,
Reagent 3 [Ecarin] (15°C): Ecarin (50 Eu/vial), verdünnt auf 0,3 EU/ml, (der Inhalt des Ecarinfläschchen wird in 5ml 0,9% NaCl-Lösung gelöst und kurz vor dem Einsatz mit einer 1:2 Mischung aus 0,9% NaCl, enthaltend 1% Prionex (Merck), und 0,1M CaCl₂-Lösung auf die Endkonzentration eingestellt.

Das Testprotokoll ist folgend wiedergegeben. Als Dil. Buffer wurde eingesetzt eine Mischung aus 16,6µl Prothrombin (gereinigt; Proteingehalt: 2,22 mg/ml) und 984 µl einer Mischung aus 900µl Tris-Puffer (0,05 M, pH 8, 37°C, + 0,1M NaCl) und 100µl Prionex (Merck).

| Testprotokoll: | Name | Ecch | |
|---|---|---|---|
| | Detector | | Chrom |
| | Start Point | | 5 sec |
| | End Point | | 180 sec |
| | Sensitivity | | Low Gain |
| | 1 SampleVol. | Zitratplasma | 5 µl |
| | Dil. Vol. | Buffer | 70 µl . |
| | 2 SampleVol. | | 0 µl |
| | ***** | | 0 µl |
| | Reagent 1 | | 30 sec |
| | Reag. Vol. | Inhib | 125 µl |
| | Rinse | | 125 µl |
| | Reagent 2 | | 120 sec |
| | Chromo | Chromo | 20 µl |
| | Rinse | | 100 µl |
| | Reagent 3 | | 210 sec |
| | Reag. Vol. | Ecarin | 20 µl |
| | Rinse | | 50 µl |

| | | | |
|---|---|---|---|
| (Rinse: 1%ige Natrium-Hypochloritiösung) | | | |

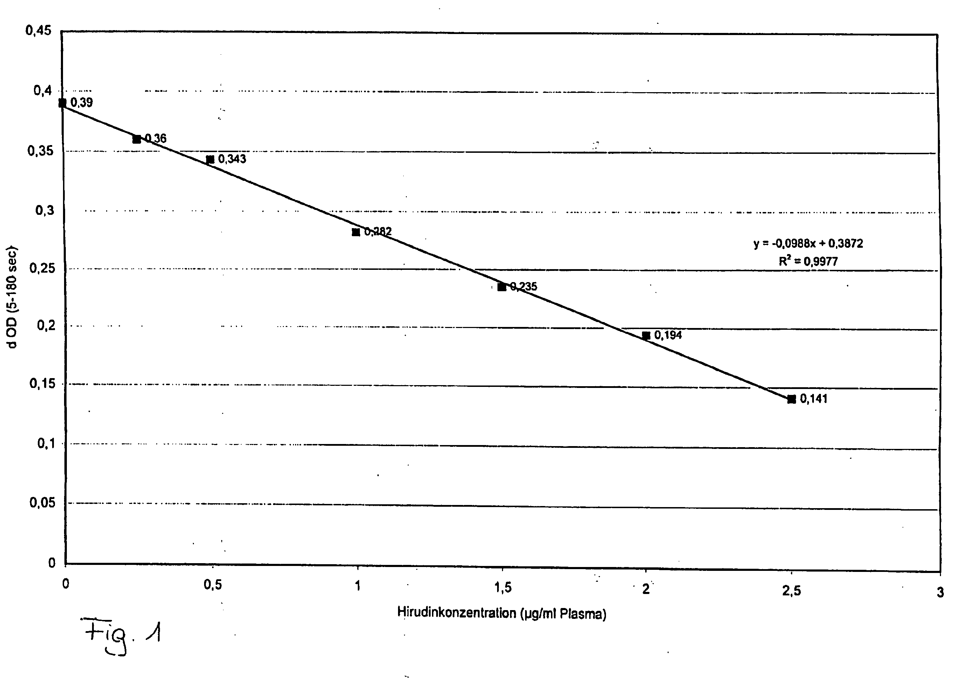

In der Fig. 1 ist die erhaltene Standardkurve wiedergegeben. Es fällt der extrem gute Korrelationskoeffizient von 0,9977 auf. Im Experiment wird lediglich die Standardprobe durch eine zu bestimmende Probe ersetzt und die unbekannte Hirudinkonzentration in der Figur 1 anhand der gemessenen Abnahme der optischen Dichte abgelesen.

## Patentansprüche

1. Verfahren zur Bestimmung der Konzentration von Thrombininhibitoren in einer nicht-trüben Körperflüssigkeit oder einem nicht-trüben Extrakt aus einer Körperflüssigkeit mit den folgenden Verfahrensschritten:
a) die Körperflüssigkeit eines Lebewesens wird erforderlichenfalls einer Abtrennung von Trübstoffen unterworfen,
b) der in Stufe a) erhaltenen nicht-trüben Körperflüssigkeit werden ein nicht in die. Umwandlung Prothrombin/aktives Meizothrombin bzw. Mtdesfg1 eingreifendes gerinnungshemmendes.Mittel, ein durch aktives Meizothrombin bzw. Mtdesfgl spaltbares chromogenes oder fluorogenes Substrat und eine Prothrombin in Meizothrombin bzw. Mtdesfgl spaltende Substanz oder Meizothrombin bzw. Mtdesfgl zugegeben, sowie, optional, Prothrombin,
c) die in Stufe b) erhaltene Lösung bzw. Mischung wird einer wellenlängenselektiven Lichtabsorptions- oder Lichtemissionsmessung in Abhängigkeit von der Zeit unterworfen,
d) aus der Verminderung der Lichtabsorption oder Lichtemission in Stufe c) je Zeiteinheit wird die in der Körperflüssigkeit enthaltene Menge des Thrombininhibitors durch Vergleich mit ermittelten Standardkurven bestimmt.

2. Verfahren zur Bestimmung der Aktivität von Thrombininhibitoren in einer nicht-trüben Körperflüssigkeit oder einem nicht-trüben Extrakt aus einer Körperflüssigkeit mit den folgenden Verfahrensschritten:
a) die Körperflüssigkeit eines Lebewesens wird erforderlichenfalls einer Abtrennung von Trübstoffen unterworfen,
b) der in Stufe a) erhaltenen nicht-trüben Flüssigkeit werden eine vorgegebene Menge an ThrombinInhibitor, ggf. ein nicht in die Umwandlung Prothrombin/aktives Meizothrombin bzw. Mtdesfgl eingreifendes gerinnungshemmendes Mittel, ein durch aktives Meizothrombin bzw. Mtdesfgl spaltbares chromogenes oder fluorogenes Substrat und eine Prothrombin in Meizothrombin bzw. Mtdesfgl spaltende Substanz oder Meizothrombin bzw. Mtdesfgl zugegeben, sowie, optional, Prothrombin,
c) die in Stufe b) erhaltene Lösung bzw. Mischung wird einer wellenlängenselektiven Lichtabsorptions- oder Lichtemissionsmessung in Abhängigkeit von der Zeit unterworfen,
d) aus der Verminderung der Lichtabsorption oder Lichtemission in Stufe c) je Zeiteinheit wird die Aktivität des Thrombininhibitors durch Vergleich mit ermittelten Standardkurven bestimmt.

3. Verfahren nach Anspruch 1 oder 2, wobei das nicht in die Umwandlung Prothrombin/aktives Meizothrombin bzw. Mtdesfgl eingreifende gerinnungshemmende Mittel ausgewählt ist aus der Gruppe "Calcium-Komplexbildner, Heparin, Heparinoide, Antithrombin III, Protein C, Fibrinpolymerisationshemmstoffe und Mischungen aus diesen Stoffen".

4. Verfahren nach einem der Ansprüche 1 bis 3, wobei die Prothrombin in Meizothrombin bzw. Mtdesfgl spaltende Substanz ausgewählt ist aus der Gruppe der Schlangengifte und Schlangengiftfraktionen.

5. Verfahren nach einem der Ansprüche 1 bis 4, wobei die Prothrombin in Meizothrombin Bzw. Mtdesfgl spaltende Substanz Ecarin ist.

6. Verfahren nach einem der Ansprüche 1 bis 5, wobei das durch aktives Meizothrombin bzw. Mtdesfgl spaltbare chromogene Substrat unter Spaltung p-Nitroanilin freisetzt und die Lichtabsorptionsmessung bei 405 nm durchgeführt wird.

7. Verfahren nach einem der Ansprüche 1 bis 6, wobei in Stufe c) eine erste Absorptions- oder Emissionsmessung nach 0 - 100 s, vorzugsweise 0 - 50, höchsvorzugsweise 5 - 15 s, und eine zweite nach anschließenden 10 - 1000 s, vorzugsweise 50 - 500 s, höchstvorzugsweise 150 - 300 s, gezählt ab Zugabe der Prothrombin in Meizothrombin bzw. Mtdesfgl spaltenden Substanz oder des Meizothrombins bzw. Mtdesfgl, durchgeführt werden.

8. Verfahren nach einem der Ansprüche 1 bis 7, wobei der Thrombininhibitor Hirudin, ein Hirolog oder ein synthetischer Thrombininhibitor ist.

9. Test Kit zur Bestimmung der Konzentration von Thrombininhibitoren in einer nicht-trüben Körperflüssigkeit oder einem nicht-trüben Extrakt aus einer Körperflüssigkeit mit folgenden Kitkomponenten: K1) einer Lösung eines nicht in die Umwandlung Prothrombin/aktives Meizothrombin bzw. Mtdesfgl eingreifenden gerinnungshemmenden Mittel, K2) einem durch aktives Meizothrombin bzw. Mtdesfgl spaltbaren chromogenen oder fluorogenen Substrat und K3) einer Lösung einer Prothrombin in Meizothrombin bzw. Mtdesfgl spaltenden Substanz, wobei die Komponente K3) ersetzt oder ergänzt sein kann durch eine Komponente K3a) einer Lösung mit Meizothrombin bzw. Mtdesfgl.

10. Test Kit nach Anspruch 9, wobei die Kitkomponente voneinander getrennt aber in einer einzigen Testkitpackung vorgesehen sind.

11. Test Kit nach einem der Ansprüche 9 oder 10, wobei als optional einsetzbare zusätzliche Kitkomponente eine Lösung mit Prothrombin vorgesehen ist.

## Claims

1. A method for determining the concentration of thrombin inhibitors in a non-turbid body liquid or in a non-turbid extract from a body liquid, comprising the following steps:
a) the body liquid of an organism is subjected, if necessary, to a separation of turbid matters,
b) to the non-turbid body liquid obtained in step a), an anti-coagulant not interfering in the conversion prothrombin/active meizothrombin or mtdesfg1, respectively, a chromogenic or fluorogenic substrate cleavable by active meizothrombin or mtdesfg1, respectively, and a substance cleaving prothrombin into meizothrombin or mtdesfg1, respectively, or meizothrombin or mtdesfg1 respectively, and, as an option, prothrombin are added,
c) the solution or mixture obtained in step b) is subjected to a wavelength-selective light absorption or light emission measurement as a function of time,
d) from the reduction of the light absorption or light emission in step c) per time unit, the quantity of the thrombin inhibitor contained in the body liquid is determined by comparison with established standard curves.

2. A method for determining the activity of thrombin inhibitors in a non-turbid body liquid or in a non-turbid extract from a body liquid, comprising the following steps:
a) the body liquid of an organism is subjected, if necessary, to a separation of turbid matters,
b) to the non-turbid liquid obtained in step a), a given quantity of thrombin inhibitor, if applicable an anti-coagulant not interfering in the conversion prothrombin/active meizothrombin or mtdesfg1, respectively, a chromogenic or fluorogenic substrate cleavable by active meizothrombin or mtdesfg1, respectively, and a substance cleaving prothrombin into meizothrombin or mtdesfg1, respectively, or meizothrombin or mtdesfg1, respectively, and, as an option, prothrombin are added,
c) the solution or mixture obtained in step b) is subjected to a wavelength-selective light absorption or light emission measurement as a function of time,
d) from the reduction of the light absorption or light emission in step c) per time unit, the activity of the thrombin inhibitor is determined by comparison with established standard curves.

3. A method according to claim 1 or 2, wherein said anti-coagulant not interfering in the conversion prothrombin/active meizothrombin or mtdesfg1, respectively, is selected from the group "calcium complex forming agents, heparin, heparinoids, antithrombin III, protein C, inhibitors of fibrin polymerization, and mixtures of these substances".

4. A method according to one of claims 1 to 3, wherein said substance cleaving prothrombin into meizothrombin or mtdesfg1, respectively, is selected from the group of snake venoms and snake venom fractions.

5. A method according to one of claims 1 to 4, wherein said substance cleaving prothrombin into meizothrombin or mtdesfg1, respectively, is ecarin.

6. A method according to one of claims 1 to 5, wherein said chromogenic substrate cleavable by active meizothrombin or mtdesfg1, respectively, releases under cleavage p-nitroaniline, and the light absorption measurement is performed at 405 nm.

7. A method according to one of claims 1 to 6, wherein in step c) a first light absorption or emission measurement is performed after 0 - 100 s, preferably 0 - 50 s, most preferably 5 - 15 s, and a second one after another 10 - 1,000 s, preferably 50 - 500 s, most preferably 150 - 300 s from the addition of said substance cleaving prothrombin into meizothrombin or mtdesfg1, respectively, or of the meizothrombin or mtdesfg1, respectively.

8. A method according to one of claims 1 to 7, wherein the thrombin inhibitor is hirudin, a hirulog or a synthetic thrombin inhibitor.

9. A test kit for determining the concentration of thrombin inhibitors in a non-turbid body liquid or in a non-turbid extract from a body liquid, comprising the following kit components: K1) a solution of an anti-coagulant not interfering in the conversion prothrombin/active meizothrombin or mtdesfg1, respectively, K2) a chromogenic or fluorogenic substrate cleavable by active meizothrombin or mtdesfg1, respectively, and K3) a solution of a substance cleaving prothrombin into meizothrombin or mtdesfg1, respectively, wherein the component K3) may be replaced or supplemented by a component K3a) of a solution with meizothrombin or mtdesfg1, respectively.

10. A test kit according to claim 9, wherein the kit components are provided separately, but in a common test kit package.

11. A test kit according to one of claims 9 or 10, wherein as an optionally usable kit component, a solution with prothrombin is provided.

## Revendications

1. Procédé pour déterminer la concentration d'inhibiteurs de la thrombine dans un liquide du corps non trouble ou un extrait non trouble d'un liquide du corps, comprenant les étapes suivantes:
a) le liquide du corps d'un organisme est soumis, si nécessaire, à une séparation de matières troubles,
b) au liquide du corps non trouble obtenu dans l'étape a), un anticoagulant n'intervenant pas dans la conversion prothrombine/meizothrombine active ou mtdesfg1, respectivement, un substrat chromogénique ou fluorogénique fissible par la meizothrombine active ou la mtdesfg1, respectivement, et une substance fissurant la prothrombine en meizothrombine ou mtdesfg1, respectivement, ou de la meizothrombine et de la mtdesfg1, respectivement, et optionnellement de la prothrombine sont ajoutés,
c) la solution ou le mélange obtenu dans l'étape b) est soumis à un mesurage de l'absorption ou de l'émission de la lumière sélectif en longueurs d'ondes en fonction du temps,
d) à partir de la réduction de l'absorption ou de l'émission de la lumière dans l'étape c) par unité de temps, la quantité de l'inhibiteur de la thrombine comprise dans le liquide du corps est calculée par comparaison avec des courbes de standard déterminées.

2. Procédé pour déterminer l'activité d'inhibiteurs de la thrombine dans un liquide du corps non trouble ou un extrait non trouble d'un liquide du corps, comprenant les étapes suivantes:
a) le liquide du corps d'un organisme est soumis, si nécessaire, à une séparation de matières troubles,
b) au liquide non trouble obtenu dans l'étape a), une quantité prédéfinie d'inhibiteur de la thrombine, le cas échéant un anticoagulant n'intervenant pas dans la conversion prothrombine/meizothrombine active ou mtdesfg1, respectivement, un substrat chromogénique ou fluorogénique fissible par la meizothrombine active ou la mtdesfg1, respectivement, et une substance fissurant la prothrombine en meizothrombine ou mtdesfg1, respectivement, ou de la meizothrombine et de la mtdesfg1 et optionnellement de la prothrombine sont ajoutés,
c) la solution ou le mélange obtenu dans l'étape b) est soumis à un mesurage de l'absorption ou de l'émission de la lumière sélectif en longueurs d'ondes en fonction du temps,
d) à partir de la réduction de l'absorption ou de l'émission de la lumière dans l'étape c) par unité de temps, l'activité de l'inhibiteur de la thrombine est calculée par comparaison avec des courbes de standard déterminées.

3. Procédé selon la revendication 1 ou 2, dans lequel ledit anticoagulant n'intervenant pas dans la conversion prothrombine/meizothrombine active ou mtdesfg1, respectivement, est choisi à partir du groupe "les complexants du calcium, l'héparine, les héparinoïdes, l'antithrombine III, la protéine C, les inhibiteurs de la polymérisation de la fibrine et les mélanges à partir de ces substances".

4. Procédé selon une des revendications 1 à 3, dans lequel ladite substance fissurant la prothrombine en meizothrombine ou mtdesfg1, respectivement est choisie à partir du groupe des venins de serpent et des fractions de venins de serpent.

5. Procédé selon une des revendications 1 à 4, dans lequel ladite substance fissurant la prothrombine en meizothrombine ou mtdesfg1, respectivement, est l'écarine.

6. Procédé selon une des revendications 1 à 5, dans lequel ledit substrat chromogénique fissible par la meizothrombine active ou la mtdesfg1, respectivement, libère sous fission la p-nitroaniline, et que le mesurage de l'absorption de la lumière est exécuté à 405 nm.

7. Procédé selon une des revendications 1 à 6, dans lequel dans l'étape c) un premier mesurage de l'absorption ou de l'émission est exécuté après 0 - 100 s, de préférence 0 - 50 s, de préférence la plus haute 5 - 15 s, et un deuxième mesurage subséquemment après 10 - 1000 s, de préférence 50 - 500 s, de préférence la plus haute 150 - 300 s, commencé lors de l'addition de ladite substance fissurant la prothrombine en meizothrombine ou mtdesfg1, respectivement, ou de la meizothrombine et de la mtdesfg1, respectivement.

8. Procédé selon une des revendications 1 à 7, dans lequel l'inhibiteur de la thrombine est l'hirudine, un hirulog ou un inhibiteur synthétique de la thrombine.

9. Kit de test pour déterminer la concentration d'inhibiteurs de la thrombine dans un liquide du corps non trouble ou un extrait non trouble d'un liquide du corps, comprenant les composants de kit suivants : K1) une solution d'un anticoagulant n'intervenant pas dans la conversion prothrombine/meizothrombine active ou mtdesfg1, respectivement, K2) un substrat chromogénique ou fluorogénique fissible par la meizothrombine active ou la mtdesfg1, respectivement, et K3) une solution d'une substance fissurant la prothrombine en meizothrombine ou mtdesfg1, respectivement, le composant K3) pouvant être remplacé ou complété par un composant K3a) d'une solution avec la meizothrombine ou la mtdesfg1, respectivement.

10. Kit de test selon la revendication 9 , dans lequel les composants du kit sont prévus séparément l'un de l'autre, mais dans un seul enveloppement.

11. Kit de test selon une des revendications 9 ou 10, dans lequel comme un composant du kit additionnel à être utilisé optionnellement, une solution avec la prothrombine est prévue.
